Europäisches Patentamt

European Patent Office

Office européen des brevets

(11) Publication number: **0 067 196**
**B1**

# EUROPEAN PATENT SPECIFICATION

(45) Date of publication of patent specification: **08.05.85**

(21) Application number: **82900165.0**

(22) Date of filing: **18.12.81**

(86) International application number:
**PCT/SE81/00383**

(87) International publication number:
**WO 82/01999 24.06.82 Gazette 82/16**

(51) Int. Cl.⁴: **A 61 M 16/06**

(54) **ANAESTHETIC MASK DEVICE.**

(30) Priority: **18.12.80 SE 8008962**

(43) Date of publication of application:
**22.12.82 Bulletin 82/51**

(45) Publication of the grant of the patent:
**08.05.85 Bulletin 85/19**

(84) Designated Contracting States:
**AT BE CH DE FR GB LI NL SE**

(56) References cited:
**SE-A-8 002 171**
**US-A-2 859 748**
**US-A-4 015 598**

(73) Proprietor: **LINDKVIST, Erik Allan**
**Korpralsvägen 38**
**S-902 53 Umea (SE)**

(72) Inventor: **LINDKVIST, Erik Allan**
**Korpralsvägen 38**
**S-902 53 Umea (SE)**

(74) Representative: **Grefberg, Lars et al**
**AWAPATENT AB Box 7402**
**S-103 91 Stockholm (SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to an anaesthetic mask device for gaseous anaesthetics, comprising a double walled cup or hood shaped part having connections for anaesthetic gas and for excess gas hose or pipe means, the edge portions of the inner and the outer walls defining a slot constituting an intake opening communicating with a passage between the walls in turn communicating with the excess gas hose or pipe means for extraction of excess gas, and adapted to engage a patient's face so as to close off said slot from both the interior of the mask and the surroundings.

During the preparations of anaesthesia as well as during the administration procedure proper in connection with surgical operations and the like an undesired leakage of anaesthetic gas takes place in the operating theatre, and especially the personnel operating near the patient's head is exposed to high concentrations of anaesthetics. When anaesthetics are to be administered to children the leakage of anaesthetic to the surrounding atmosphere will be still higher because, in order to get the child used to accept the anaesthetic mask, it is often necessary to apply the mask to the child and lift it away repeatedly, which results in large amounts of anaesthetics flowing out. Although this problem has per se been observed for a long time no useful solution has been found. Neither an improved general ventilation of the operating theatre nor a concentrated air extraction, local extraction, at the head of the operating-table have given the desired result.

It has earlier been proposed that the anaesthetic mask should be provided with a sealing device working under vacuum and sucking the mask fast to the patient's face to prevent leakage of anaesthetic gas, and to combine such a device with a valve device provided in the anaesthetic gas supply line to shut off the supply of gas as soon as the mask is removed from the patient's face and the vacuum is eliminated.

However, means for the manipulation of the anaesthetic gas flow are regarded as risky and can, especially in combination with vacuum devices, be dangerous to the patients.

Sucking the mask to the patient's face may make the patient feel uncomfortable and especially so when it is a matter of children who cannot always understand the reasons for the steps taken in connection with the administration of anaesthetics.

It has also earlier been suggested in US—A—4015598 to provide an anaesthetic mask hood or cup with an inner and an outer wall forming between same a passage open towards the patient's face and connected to a suction device. With the known mask any anaesthetic gas escaping below the seal forming edge of the inner wall when the mask is applied towards the patient's face is intended to be sucked into the passage between the walls towards the excess hose or the like. The known mask device has two pairs of hose or pipe connections protruding sideways from the mask hood or cup, one pair to be connected to the anaesthetic gas source opening out in the interior of the mask, and one pair to be ending in said passage and connected to the suction device. The former pair of connections end in ejection openings one at each side of the interior of the mask, the two converging anaesthetic gas streams entering through said openings forming a resulting stream passing through the center portion of the interior towards the patient's face. Upon removing the mask from the patient's face the anaesthetic gas stream will flow freely through the mask, without being sucked away through the passage between the walls of the mask hood.

A desire expressed by experts in the field is that devices for the removal of leaking or otherwise escaping anaesthetic gas should be constructed in such a way that they require neither any change of prevailing anaesthetic administration routines nor any special measures to function. Another desire is that it should be possible to use existing control equipment without changes. Thus it is a main object of the invention to provide a device preventing unintentional spreading of anaesthetic gas outside the anaesthetic mask.

The invention is characterised in that the upper portion of the double walled cup is relatively rigid whereas the seal forming edge or border portions at both the inner and outer wall are of soft yielding material, that a connection means is centrally located at the upper portion of the cup or hood shaped part, said means including an inner channel communicating with the interior of the cup or hood shaped part and an outer channel communicating with the passage between the walls, and that a deflecting means is arranged inside the cup or hood shaped parts adjacent and opposite to the inner channel of the connection means and adapted to deflect the anaesthetic gas entering the cup or hood shaped part through said central channel towards and along the inside thereof to facilitate the collection of gas flowing freely out from the mask.

Embodiments of the device according to the invention will be described more fully below by way of example, reference being made to the accompanying drawings, in which:

Fig. 1 is cross-sectional view of an embodiment of an anaesthetic mask according to the invention;

Fig. 2 is likewise a sectional view of a preferred embodiment of the device.

The drawings comprise only such details as are essential to the comprehension of the invention and, since the design of the anaesthetic mask proper can vary to a high degree according to manufacturer, anaesthetic system and size, the mask has only been schematically illustrated in two principal embodiments.

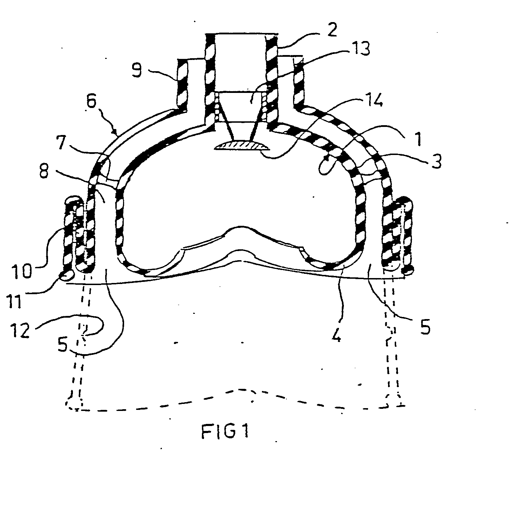

The anaesthetic mask proper, generally designated by 1, comprises a connection piece 2 from which the cup- or hood-shaped main part 3 extends. In the embodiment according to Fig. 1 said main part merges in an inward-bent, thinner edge portion which forms a sealing border against the patient's chin, cheeks and nose ridge.

According to the invention an extraction slot 5 is arranged around at least the edge portion of the anaesthetic mask. In the embodiment according to Fig. 1 this slot is formed by another hood-shaped means 6 being arranged on the outside of the mask proper 1.

Said means 6 may be connected with the mask 1 by studs or the like 7 which constitute spacing means and keep passages 8 from the slot 5 at the lower edge of the mask open to a connection piece 9 which preferably is coaxial with the piece 2 and to which a sucking line is connected.

The body of the hood-shaped means 6 may like the mask 1, be of relatively rigid construction. In the embodiment according to Fig. 1 the body of the means 6 merges in a collar 10 made of thinner material and terminating in an edge bead 11. Folding notches 12 are formed in the collar 10 by attenuation of material or in some other way so that the collar 10, which normally takes the position indicated by dash lines, is apt to fold up to the position shown Fig. 1 when it meets the patient's face.

Fitted in a nipple 13, inserted in the connection piece 2 of the anaesthetic mask, 1 is a spreader disk 14 for distribution of inflowing anaesthetic mixture along the inside of the mask so that the gas flow is forced to spread conically within the mask instead of flowing straight out from the nipple as is the case with prior art masks.

The connection piece 9 of the extraction means 6 is coupled to the evacuation or suction line. As long as the mask is kept free, outflowing anaesthetic gas will be extracted from the space delimited by the collar 10 in unfolded position. The extraction continues also while the mask is being drawn towards the patient's face and is interrupted when the border 4 of the anaesthetic mask seals against the face. Any anaesthetic gas leaking out along the border 4 is extracted all the time the mask is kept applied. When the mask is removed from the patient the collar 10 unfolds against and functions as before. To prevent too high a negative pressure from arising in the extraction space around the mask, which might have as a result that the mask would be sucked fast to the patient, the extraction line is provided with a valve means sensing the negative pressure and keeping the negative pressure in said space within an appropriate value adapted to the function.

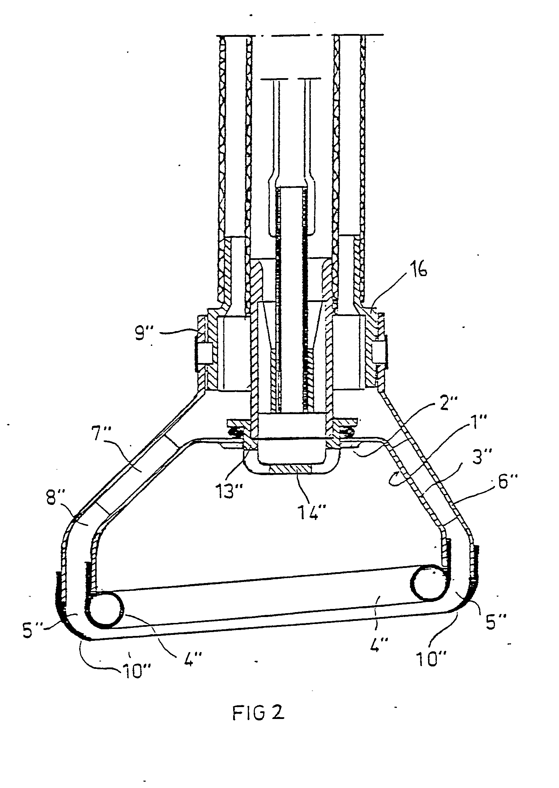

The mask 1" shown in Fig. 2 comprises, like the mask described above, an inner part 3" and an outer part 6" between which an extraction passage 8" is formed. In this embodiment the parts 3" and 6" are made of comparatively rigid, crystal-clear material and have generally parallel portions making it possible continuously to watch the patient's face and especially his lips without making it necessary to lift away the mask.

The inner part 3" is in this case provided with a soft seal 4", preferably in the form of a closed, tube-like profile, instead of the above-mentioned sealing borders.

The outer part 6" has a circumferentially extending soft strip 10" tapering towards the free edge of the part 6" and bent in inward direction when not acted upon, and this strip is adapted to bend towards the sealing profile 4" when it is applied to the patient's face, thereby reducing or preventing extraction through the slot 5". Also in this case the suction source may be provided with control means preventing buildup of too high negative pressure.

The inner part 3" of the mask has a central opening surrounded by a bead 2" and entering through this bead is the end of a double nipple 13" to which coaxial lines for anaesthetic gas and exhalation air are connected. The inner end of the nipple 13" is formed as a spreader disk 14" making the anaesthetic gas flow out substantially radially from the nipple end. The gas will thus sweep along the walls in the mask, which facilitates collection through the slot 5".

The nipple 13" is combined with an outer nipple 16 to which the connection piece 9" of the evacuation line is connected.

As a rule the adjustment of the anaesthetic gas flow takes place with the mask lifted off the patient, while the outflowing gas is spread in the mask, is collected in the suction slot 5" and removed through the passage 8". When the mask is applied to the patient the thin strip 10" will bend inwards, whereby it reaches the sealing profile 4" and prevents extraction of gas from the interior of the mask. If some part of the mask is withdrawn from the face of the patient then the strip withdraws from the profile to allow extraction. As soon as the mask is entirely removed from the patient the suction slot opens in its entirety, whereby gas is prevented from escaping into the surrounding atmosphere.

Thus, in the embodiments herein described, extraction of anaesthetic gas continues all the time the mask is removed from the patient and any gas that would otherwise have leaked out due to unsatisfactory application along some portion of the mask is collected and extracted when the mask has been applied to the patient the extraction is interrupted and the mask functions entirely as a conventional mask. The device according to the invention can thus be used in an entirely conventional manner and as far as the operating personnel is concerned it requires no additional steps at all for functioning.

The invention must not be considered limited to that described in the foregoing and shown in the drawings but can be modified in various ways within the scope of the appended claims.

**Claims**

1. Anaesthetic mask device for gaseous anaesthetics, comprising a double walled cup or hood shaped part (3) having connections for anaesthetic gas and for excess gas hose or pipe means, the edge portions of the inner and the outer walls defining a slot constituting an intake opening (5, 5″) communicating with a passage between the walls in turn communicating with the excess gas hose or pipe means for extraction of excess gas, and adapted to engage a patient's face so to close off said slot from both the interior of the mask and the surroundings, characterized in that the upper portion of the double walled cup is relatively rigid whereas the seal forming edge or border portions (4, 4″ and 10, 10″) at both the inner and the outer wall (1, 1″ and 6, 6″) are of soft unyielding material, that a connection means (2, 2″, 9, 9″) is centrally located at the upper portion of the cup or hood shaped part, said means including an inner channel communicating with the interior of the cup or hood shaped part and an outer channel communicating with the passage (8, 8′) between the walls, and that a deflecting means (14, 14″) is arranged inside the cup or hood shaped part adjacent and opposite to the inner channel of the connection means and adapted to deflect the anaesthetic gas entering the cup or hood shaped part through said central channel towards and along the inside thereof to facilitate the collection of gas flowing freely out from the mask.

2. Device as claimed in claim 1, characterized in that at least the upper portion of the double walled cup or hood shaped part is of transparent or clear material.

3. Device as claimed in claim 1, characterized in that the connection means (2, 2″, 9, 9″) is adapted to receive a pipe or hose means having an end piece with concentrically arranged channels corresponding to the outer and inner channels and the connecting means.

4. Device as claimed in claim 1, characterized in that means is provided for controlling the suction or negative pressure in the slot shaped opening (5, 5″) and that said means reduces the suction effect when the slot shaped opening is closed by the application of the mask to the patient's face.

**Revendications**

1. Masque d'anesthésie destiné à des anesthésiques gazeux, comprenant une partie (3) à double paroi en forme de capot ou de coupelle, ayant des raccords pour des canalisations souples ou non d'anesthésiques gazeux et de gaz en excès, les parties marginales des parois interne et externe délimitant une fente constituant une ouverture d'entrée (5, 5″) communiquant avec un passage formé entre les parois et qui communique à son tour avec la canalisation souple ou non de gaz en excès afin que le gaz en excès soit évacué, et destiné à coopérer avec la face d'un patient avec fermeture de ladite fente à la fois par rapport à l'intérieur du masque et par rapport à l'atmosphère environnante, caractérisé en ce que la partie supérieure de la coupelle à double paroi est relativement rigide alors que les parties marginales ou des bords (4, 4″, 10, 10″) formant joints d'étanchéité au niveau de la paroi interne et de la paroi externe (1, 1″ et 6, 6″) sont formées d'un matériau mou et souple, en ce qu'un dispositif de raccordement (2, 2″, 9, 9″) est placé au centre dans la partie supérieure de la partie en forme de capot ou de coupelle, ce dispositif comprenant un canal interne communiquant avec l'intérieur de la partie en forme de capot ou de coupelle et un canal externe communiquant avec le passage (8, 8′) formé entre les parois, et en ce qu'un dispositif de déviation (14, 14″) est disposé à l'intérieur de la partie en forme de capot ou de coupelle près du canal interne du dispositif de raccordement et en face de celui-ci, et est destiné à dévier l'anesthésique gazeux pénétrant dans la partie en forme de capot ou de coupelle par l'intermédiaire du canal central vers l'intérieur et le long de cette partie afin que la collecte du gaz s'écoulant librement hors du masque soit facilitée.

2. Masque selon la revendication 1, caractérisé en ce que la partie supérieure au moins de la partie à double paroi en forme de capot ou de coupelle est formée d'une matière transparente ou limpide.

3. Masque selon la revendication 1, caractérisé en ce que le dispositif de raccordement (2, 2″, 9, 9″) est destiné à loger une tuyauterie souple ou non, ayant un organe d'extrémité comportant des canaux formés concentriquement et correspondant aux canaux externe et interne et au dispositif de raccordement.

4. Masque selon la revendication 1, caractérisé en ce qu'un dispositif est destiné à régler la pression d'aspiration ou la dépression dans l'ouverture en forme de fente (5, 5″) et en ce que ce dispositif réduit l'effet d'aspiration lorsque l'ouverture en forme de fente est fermée par application du masque sur la face du patient.

**Patentansprüche**

1. Narkosemaske für Anästhesiegas mit einem doppelwandigen, tassen- oder haubenförmigen Teil (3) mit Anschlüssen für Anästhesiegas- und Überschußgasschlauch oder -leitung, wobei die Randteile der inneren und der äußeren Wand eine Spalt bilden, der eine Einsaugöffnung (5, 5″) bildet, die mit einer Passage zwischen den Wänden kommuniziert, welche ihrerseits mit den Überschußgasschlauch zum Absaugen von überschüssigem Gas in Verbindung steht, wobei die Maske zum Anlegen auf das Gesicht eines Patienten vorgesehen ist und dadurch der genannte Spalt sowohl gegenüber dem Inneren der Maske als auch der Umgebung abgeschlossen wird, dadurch gekennzeichnet, daß der obere Teil der doppelwandigen Maske verhältnismäßig steif ist, wogegen die als Dichtung dienenden Lippen oder Randteile (4, 4″ und 10, 10″) sowohl an der Innenwand als auch der Außenwand (1, 1″ und 6, 6″) aus weichem, nachgiebigem Material bestehen, daß ein Verbindungsstück (2, 2″, 9, 9″) mittig am Oberteil der Maske angebracht ist und einen Innenkanal einschließt, der mit dem Inneren der Maske kommuniziert, sowie einen äußeren Kanal, der mit der Passage (8, 8′) zwischen den Wänden kommuniziert, sowie daß ein Ablenkungsorgan (14, 14″) an der Innenseite der Maske vor oder gegenüber dem inneren Kanal des Verbindungsstückes angebracht ist und dazu dient, das in die Maske durch den genannten inneren Kanal einströmende Anästhesiegas zur und entlang der Innenseite der Maske umzulenken, um das Auffangen von frei aus der Maske ausströmendem Gas zu erleichtern.

2. Vorrichtung nach Anspruch 1, dadurch gekennzeichnet, daß wenigstens der obere Teil des doppelwandigen tassen- oder haubenförmigen Teils aus transparentem oder klarem Material besteht.

3. Vorrichtung nach Anspruch 1, dadurch gekennzeichnet, daß das Verbindungsstück (2, 2″, 9, 9″) dazu vorgesehen ist, ein Rohr- oder Schlauchorgan aufzunehmen, das ein Endstück mit konzentrisch angebrachten Kanälen entsprechend dem äußeren und inneren Kanal und dem Verbindungsstück aufweist.

4. Vorrichtung nach Anspruch 1, dadurch gekennzeichnet, daß Mittel zur Steuerung des Absaugens oder Unterdruckes in der schlitzartigen Öffnung (5, 5″) vorgesehen sind, und daß die genannten Mittel die Saugwirkung verringern, wenn die spaltförmige Öffnung durch Aufsetzen der Maske auf das Gesicht eines Patienten geschlossen wird.

0 067 196

FIG 1

1

FIG 2